(19) Europäisches Patentamt
European Patent Office
Office européen des brevets

(11) Numéro de publication: **0 012 051**
**A1**

(12) # DEMANDE DE BREVET EUROPEEN

(21) Numéro de dépôt: 79400838.3

(22) Date de dépôt: 09.11.79

(51) Int. Cl.³: C 07 D 239/96
C 07 D 401/04, C 07 D 403/04
C 07 D 413/04, A 61 K 31/505

(30) Priorité: 27.11.78 FR 7833438

(43) Date de publication de la demande: 11.06.80 Bulletin 80/12

(84) Etats Contractants Désignés: DE NL SE

(71) Demandeur: Société dite: BERRI-BALZAC
11 bis Rue Balzac
F-75008 Paris(FR)

(72) Inventeur: Baronnet, René
15 bis Rue Henri Regnault
F-92300 Garches(FR)

(72) Inventeur: Callendret, Raymond
1 bis Rue Bellavoine
F-78230 Le Pecq(FR)

(74) Mandataire: Polus, Camille et al,
c/o Cabinet Lavoix 2, Place d'Estienne d'Orves
F-75441 Paris Cedex 09(FR)

(54) **Dérivés d'amino-3 dioxo-2,4 (1H,3H) quinazolines, leur procédé de préparation et composition thérapeutique les contenant.**

(57) L'invention a pour objet des composés de formule:

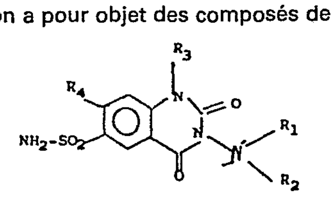

dans laquelle $R^1$ représente l'hydrogène ou un groupe alkyle en $C_1$ à $C_5$ et $R_2$ représente un group alkyle inférieur, ou bien $R_1$ et $R_2$ forment ensemble et avec l'atome d'azote auquel ils sont fixés un hétérocycle à 5 ou 6 chaînons pouvant contenir un autre hétéroatome (tel que l'oxygène ou l'azote); $R_3$ représente l'hydrogène ou un groupe alkyle inférieur et $R_4$ représente l'hydrogène ou un atome d'halogène ; la forme tautomère de type amino-3- hydroxy-2 (3,H) quinazoline-4 des composés de formule I dans lesquels $R_3$ est l'hydrogène ; et les sels d'addition avec des acides de composés ci-dessus.

Ces nouveaux composés ont notamment une activité diurétique.

EP 0 012 051 A1

Croydon Printing Company Ltd.

Dérivés d'amino-3 dioxo-2,4 (1H,3H) quinazolines, leur procédé de préparation et composition thérapeutique les contenant.-

Dérivés d'amino-3 dioxo-2,4 (1H,3H) quinazolines, leur procédé de préparation et composition thérapeutique les contenant.

La présente invention a pour objet de nouveaux dérivés d'amino-3 sulfamido-6 (1H, 3H) quinazoline diones-2,4, un procédé pour les préparer et leurs applications en thérapeutique, notamment comme diurétiques.

Dans Chemical Abstracts, Vol 79 (1973) p.457, 18.752n et SP 7301674 on a déjà décrit des sulfonamides de formule

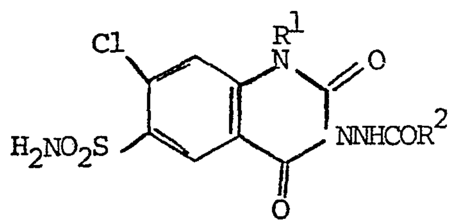

qui possèdent une activité diurétique.

La présente invention se propose de fournir de nouveaux dérivés sulfamidiques ayant des propriétés améliorées.

Les nouveaux composés de l'invention répondent à la formule :

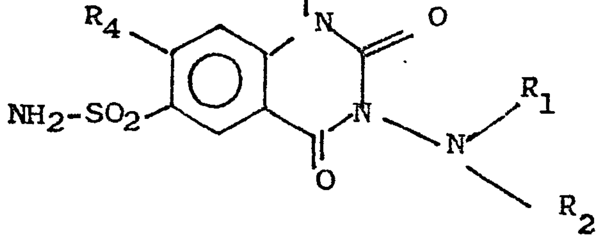

(I)

dans laquelle $R_1$ représente l'hydrogène ou un groupe alkyle en $C_1$ à $C_5$ et $R_2$ représente un groupe alkyle inférieur, ou bien $R_1$ et $R_2$ forment ensemble et avec l'atome

d'azote auquel ils sont fixés un hétérocycle à 5 ou 6 chaînons pouvant contenir un autre hétéroatome (tel que l'oxygène ou l'azote) ; $R_3$ représente l'hydrogène ou un groupe alkyle inférieur et $R_4$ représente l'hydrogène ou un atome d'halogène.

Les groupes alkyle inférieurs comportent par exemple de 1 à 4 atomes de carbone.

L'invention comprend aussi les sels d'addition des composés ci-dessus portant un groupe suffisamment basique avec des acides (notamment l'acide chlorhydrique, l'acide bromhydrique, l'acide sulfurique, etc...)

Dans le cas où $R_3$ représente l'hydrogène, les nouveaux dérivés sont susceptibles d'exister sous la forme tautomère amino-3 hydroxy-2 (3,H) quinazoline-4 de formule :

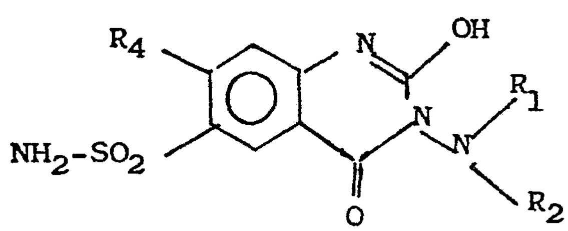

(Ia)

et l'invention comprend aussi cette forme tautomère.

Ces nouveaux dérivés sont doués de propriétés salidiurétiques et peuvent être utilisés comme principes actifs de nouveaux médicaments diurétiques.

Parmi les dérivés de formule I, ceux dans lesquels $R_3$ est un atome d'hydrogène et $R_4$ un atome d'halogène (notamment de chlore) sont particulièrement intéressants pour leurs propriétés salidiurétiques.

L'invention a encore pour objet un procédé de préparation des composés précités, caractérisé en ce qu'on fait réagir de l'acide chlorosulfonique sur une amino-3(1H,3H) dioxo-2,4 quinazoline de formule

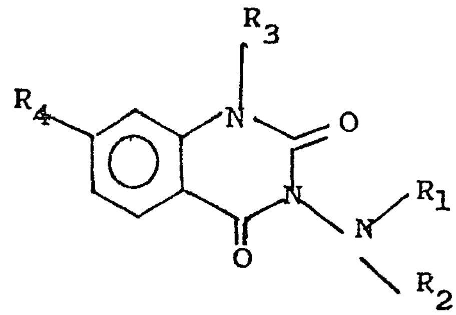

(II)

dans laquelle $R_1$, $R_2$, $R_3$ et $R_4$ ont les significations précitées, obtenant ainsi un composé de formule

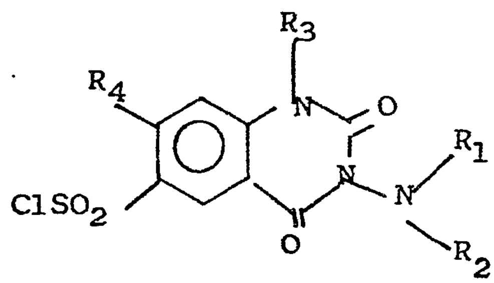

(III)

dans laquelle $R_1$, $R_2$, $R_3$ et $R_4$ ont les significations précitées, que l'on fait ensuite réagir avec de l'ammoniac pour obtenir le composé de formule (I).

Les composés de départ de formule II peuvent être obtenus en suivant le schéma réactionnel ci-après, les symboles $R_1$, $R_2$, $R_3$ et $R_4$ ayant les significations données pour la formule (I).

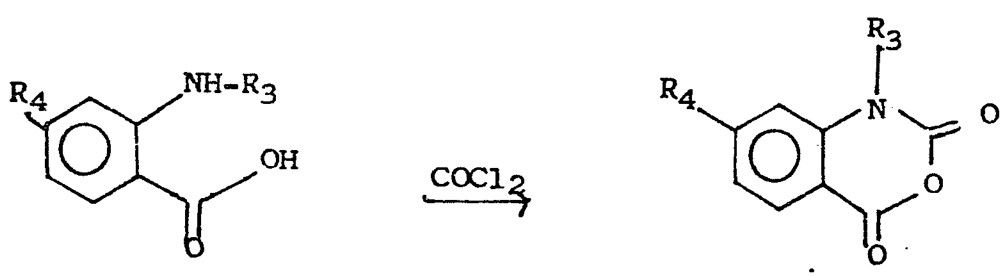


(IV) ( V )

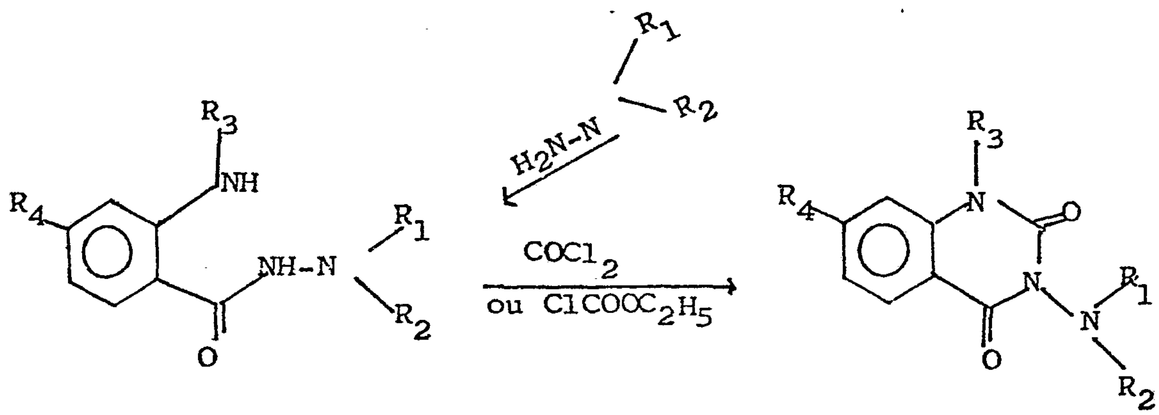


(VI) (II)

Les acides anthraniliques de formule (IV) traités par le chlorure de carbonyle donnent des acides isatoïques de formule (V), lesquels sous l'action des hydrazines $H_2N-N\langle^{R_1}_{R_2}$ donnent les hydrazides anthraniliques de formule (VI) qui sont cyclisés par $COCl_2$ ou $ClCOOC_2H_5$ en les quinazoline diones de formule (II).

On peut mettre le procédé en oeuvre de la façon suivante :

1) Procédé de préparation des dioxo-2,4(1H,3H) quinazolines de formule (II) :

a) Préparation de l'anhydride isatoïque de formule (V) :

L'acide anthranilique (IV) convenablement substitué est dissous dans une solution aqueuse d'acide chlorhydrique ou éventuellement dans le dioxane lorsque cet acide n'est pas soluble en milieu acide. On fait passer dans cette solution un courant de chlorure de carbonyle (phosgène). L'anhydride isatoïque (V) précipite peu à peu. Après 1 h 30 de contact, le produit est essoré, rincé à l'eau et séché à l'étuve à 100 °C. Les eaux-mères peuvent subir l'action d'un deuxième contact avec le phosgène pour fournir un autre jet de cristallisation.

b) Préparation de l'hydrazide anthranilique de formule (VI) :

A une suspension dans un alcool aliphatique, ou mieux dans un éther alcoylique de l'éthylène glycol notamment l'éther diméthylique, de 1 mole d'anhydride isatoïque (V), on ajoute 1 mole d'hydrazine $H_2N-N\langle^{R_1}_{R_2}$. On laisse le mélange en réaction jusqu'à cessation de dégagement d'anhydride carbonique. Le solvant est partiellement éliminé sous pression réduite et le produit de réaction est généralement cristallisé par addition de benzène. La pureté du produit est suffisante pour l'étape ultérieure.

c) Cyclisation des hydrazides anthraniliques (VI) en dioxo-2,4(1H,3H) quinazolines de formule (II) :

- Méthode A :

On dissout dans le minimum d'acide chlorhydrique 2N l'hydrazide (VI). La solution est alors traitée au chlorure de carbonyle. Le produit de réaction précipite lentement dans le milieu. Après 1 h de contact, le produit est essoré, rincé à l'eau et séché. Il est purifié par recristallisation dans l'éthanol ou l'isopropanol.

- Méthode B :

L'hydrazide (VI) est cyclisé en amino-3 dioxo-2,4 (1H,3H) quinazoline (II) en chauffant sous reflux le produit en présence de chloroformiate d'éthyle pendant 3 à 4 h. L'excès de chloroformiate d'éthyle est éliminé par distillation et le résidu est cristallisé à partir de l'éthanol.

2) Procédé de préparation des composés de formule (I) suivant l'invention :

a) Sulfonation des dioxo-2,4 (1H,3H) quinazolines (II) en dérivés chlorosulfonés de

formule (III) :

La sulfonation des dioxo-2,4 (1H,3H) quinazolines (II) est effectuée avantageusement par chauffage en présence d'acide chlorosulfonique pendant 3 heures à 100 °C. Le dérivé chlorosulfoné (III) obtenu est, après refroidissement du mélange, précipité sur de la glace et essoré. Le produit brut est utilisé directement pour la préparation du sulfonamide.

b) Préparation des dioxo-2,4 (1H,3H) quinazolines sulfonamidées en 6 de formule (I) :

Le dérivé sulfonamidé est obtenu à partir du sulfochlorure précédent, par traitement à l'ammoniac, de préférence une solution aqueuse concentrée d'ammoniaque.

Les préparations et exemples non limitatifs suivants illustrent le procédé de l'invention.

Préparation 1

Préparation de l'anhydride chloro-7 isatoïque

15 g d'acide chloro-4 anthranilique sont dissous à chaud dans 300 $cm^3$ de dioxane. On fait passer à travers la solution agitée et refroidie à 40°C un courant de phosgène pendant 1 h 30 ; le milieu réactionnel est purgé au moyen d'un courant d'air. Le produit de réaction, apparu par refroidissement, est essoré. Les eaux-mères fournissent un deuxième jet par concentration . F déc. = 290 °C.

Préparation 2

Préparation de l'amino-2 chloro-4 N-diméthylamino benzamide

15 g d'anhydride chloro-7 isatoïque sont placés en suspension dans 100 $cm^3$ d'éthanol porté à 60 °C. On ajoute lentement 5 g de diméthyl-1,1 hydrazine. Après addition, la température est maintenue jusqu'à cessation de dégagement d'anhydride carbonique. L'éthanol est alors éliminé sous pression réduite et le produit de réaction est recristallisé dans du benzène.

F = 128 - 130 °C.

Préparation 3

Préparation du chloro-4 N-diméthylamino méthylamino-2 benzamide

A 21 g d'anhydride chloro-7 méthyl-1 isatoïque placés en suspension dans 100 $cm^3$ de diméthoxy-1,2 éthane porté à une température de 40 °C, on ajoute lentement 66 g de N,N-diméthylhydrazine. On maintient ainsi en réaction jusqu'à cessation du dégagement d'anhydride carbonique. La concentration à demi-volume permet de cristalliser le produit de réaction qui peut être recristallisé dans un mélange benzène-cyclohexane. F = 146 °C.

Préparation 4

Préparation de la chloro-7 diméthylamino-3 dioxo-2,4 (1H, 3H) quinazoline (cyclisation au phosgène)

9 g d'amino-2 chloro-4 N-diméthylamino benzamide sont dissous dans 250 $cm^3$ d'acide chlorhydrique 2 N. A travers la solution, on fait passer un courant de phosgène pendant 1 heure. Après avoir purgé l'appareil, le produit qui a précipité est essoré et lavé à l'eau. On obtient un deuxième jet de produit en faisant passer un nouveau courant de phosgène dans les eaux-mères. On purifie le produit par cristallisation dans l'éthanol.

F = 300-302 °C.

Préparation 5

Préparation de la diméthylamino-3 dioxo-2,4 (1H,3H) quinazoline (cyclisation au phosgène)

25,6 g d'amino-2 N-diméthylamino benzamide sont mis en solution dans 500$cm^3$ d'acide chlorhydrique 2N. A travers la solution, on fait passer un courant de phosgène pendant 1 h 30 ; après avoir purgé l'appareillage, le produit précipité est essoré, lavé à l'eau et séché. Il est purifié par recristallisation dans l'éthanol. F = 234 °C.

Préparation 6

Préparation de la dioxo-2,4 méthyl-1 pipéridino-3(1H,3H) quinazoline (cyclisation au phosgène)

10 g de méthylamino-2 N-pipéridino benzamide sont mis en solution dans 200 $cm^3$ d'acide chlorhydrique 2 N. On fait passer à travers la solution agitée un courant de phosgène pendant 1 heure. Après avoir purgé l'appareillage, la solution est amenée à pH 4 par addition de lessive de soude. Le précipité obtenu est essoré et purifié par recristallisation dans l'éthanol. F = 164 °C.

Préparation 7

Préparation de la chloro-7 éthyl-1 dioxo-2,4 morpholino-3 (1H,3H) quinazoline (cyclisation au chloroformiate d'éthyle)

6 g de chloro-4 éthylamino-2 N-morpholinobenzamide sont chauffés sous reflux pendant 3 heures dans 20 $cm^3$ de chloroformiate d'éthyle. Après refroidissement partiel, l'excès de chloroformiate d'éthyle est éliminé par distillation sous pression réduite. Le résidu est alors repris par l'éthanol pour être cristallisé. F = 240 °C.

Exemple 1

a) Préparation du [chloro-7 diméthylamino-3 dioxo-2,4 (1H,3H) quinazolinyl-6 ] sulfochlorure.

9,5 g de chloro-7 diméthylamino-3 dioxo-2,4 (1H,3H) quinazoline sont ajoutés par petites portions à 35 $cm^3$ d'acide chlorosulfonique. Le mélange est chauffé à 100 °C au moyen d'un bain d'huile pendant 3 heures. Après refroidissement, le mélange est versé lentement et avec précaution sur 200 g de glace pilée. Le sulfochlorure précipité est essoré et rincé avec 30 $cm^3$ d'eau. La pureté du produit ainsi obtenu est suffisante pour l'étape ultérieure.

b) Préparation de la chloro-7 diméthylamino-3 dioxo-2,4

(1H,3H) sulfonamido-6 quinazoline

9,5 g de [chloro-7 diméthylamino-3 dioxo-2,4 (1H,3H) quinazolinyl-6 ] sulfochlorure sont chauffés dans 100 $cm^3$ d'ammoniaque concentrée pendant 1 heure. Le produit cristallise par concentration de la solution. Il est essoré, rincé à l'eau et recristallisé dans de l'éthanol. F = 235-238 °C (260-270 °C)

Exemple 2

Préparation de la méthyl-1 chloro-7 diméthylamino-3 dioxo -2,4 sulfonamido-6 (1H,3H) quinazoline

5 g de méthyl -1 chloro-7 diméthylamino-3 dioxo-2,4 (1H,3H) quinazoline sont introduits par petites portions et sous agitation dans 20 $cm^3$ d'acide chlorosulfonique. Après la fin de l'addition, la solution obtenue est chauffée à 100 °C pendant 3 heures. Après refroidissement, la solution est versée lentement et avec précaution sur 100 g de glace pilée. Le produit de réaction précipite, il est essoré, rincé avec 10 $cm^3$ d'eau glacée. Le produit brut ainsi obtenu est chauffé dans 50 $cm^3$ d'ammoniaque concentrée pendant 1 heure. Le produit de réaction précipite complètement par refroidissement. Il est alors essoré et purifié par cristallisation dans l'éthanol.

F = 292 °C

Exemple 3

Préparation de la diméthylamino-3 dioxo-2,4 (1H,3H) sulfonamido-6 quinazoline

6 g de diméthylamino-3 dioxo-2,4(1H,3H) quinazoline sont ajoutés par petites portions à 25 $cm^3$ d'acide chlorosulfonique. Le mélange est chauffé à 100 °C au moyen d'un bain d'huile pendant 3 heures. Après refroidissement, le mélange est versé lentement et avec précaution sur 200 g de glace pilée. Le (diméthylamino-3 dioxo-2,4 (1H,3H) quinazolinyl-6) sulfochlorure précipite. Il est essoré et rincé avec 20 $cm^3$ d'eau. Le produit ainsi obtenu est

chauffé dans 50 $cm^3$ d'ammoniaque concentrée pendant 1 heure. Le produit cristallise par neutralisation. Il est essoré, rincé à l'eau et cristallisé dans l'éthanol. F = 305 °C.

Exemple 4

Préparation de la méthyl-1 diméthylamino-3 dioxo-2,4 (1H,3H) sulfonamido-6 quinazoline

6 g de méthyl-1 diméthylamino-3 dioxo-2,4 (1H,3H) quinazoline sont ajoutés par petites portions à 30 $cm^3$ d'acide chlorosulfonique. Le mélange est chauffé à 100 °C au moyen d'un bain d'huile pendant 3 heures. Après refroidissement, le mélange est versé lentement et avec précaution sur 200 $cm^3$ de glace pilée. Le (méthyl-1 diméthylamino-3 dioxo-2,4 (1H,3H) quinazolinyl-6) sulfochlorure précipite. Il est essoré et rincé avec 20 $cm^3$ d'eau. Le produit ainsi obtenu est chauffé dans 50 $cm^3$ d'ammoniaque concentrée pendant 1 heure. Après refroidissement, le produit obtenu est essoré. Il est purifié par recristallisation dans l'eau. F = 263-265 °C.

Par des modes opératoires analogues à ceux décrits aux exemples précédents on a préparé :

la chloro-7 morpholino-3 dioxo-2,4 (1H,3H) sulfonamido-6 quinazoline, F = 312 °C.

la méthyl-1 chloro-7 morpholino-3 dioxo-2,4 (1H,3H) sulfonamido-6 quinazoline, F = 280 °C

l'éthyl-1 chloro-7 diméthylamino-3 dioxo-2,4 (1H,3H) sulfonamido-6 quinazoline, F = 237 °C

la chloro-7 diéthylamino-3 dioxo-2,4 (1H,3H) sulfonamido-6 quinazoline, F = 150 °C

la chloro-7 pipéridino-3 dioxo-2,4 (1H,3H) sulfonamido-6 quinazoline, F = 277 °C

la méthyl-1 chloro-7 pipéridino-3 dioxo-2,4 sulfonamido-6 quinazoline, F = 295 °C

et la chloro-7 N-méthylpipérazino-3 dioxo-2,4 sulfonamido-6 quinazoline, F = 316 °C

L'invention a également pour objet une composition thérapeutique ayant notamment une activité diurétique, caractérisée en ce qu'elle contient, à titre de principe actif, un composé de formule (I) ou (Ia) précitée ou un sel d'addition avec un acide thérapeutiquement administrable de celui-ci, associé à un véhicule pharmaceutiquement acceptable.

Les résultats d'essais toxicologiques et pharmacologiques rapportés ci-dessous illustrent les activités des composés de l'invention.

A - Toxicité

Les composés de formules (I) et (Ia) sont des substances dont la toxicité aiguë sur la souris est faible : elle est supérieure à 1 g/kg par voie I.P. et supérieure à 2,5 g/kg par voie orale.

Ces produits, en application sub-aiguë, ne donnent pas d'altération anatomique du nephron.

B - Activité pharmacologique

Les composés de formules (I) et (Ia) présentent essentiellement une action salidiurétique et sont dénués d'actions secondaires susceptibles de nuire à leur utilisation.

Certains d'entre eux peuvent être utilisés, non seulement par voie orale, mais aussi par voie injectable où ils sont capables de provoquer une abondante diurèse immédiate.

Ils ont été comparés à deux substances témoins : le furosémide (acide chloro-4 furfurylamino-2 sulfamoyl-5 benzoïque) et l'hydrochlorothiazide (dioxo-1,1 chloro-6 dihydro-3,4 sulfamoyl-7 (1,2,4)H benzothiadiazine).

L'étude de l'activité diurétique a été effectuée comme suit :

l'expérience a été réalisée en utilisant la technique de LIPSCHITZ & Coll. (J. Pharm. Exp. Ther. 1943, 79, p. 97) modifiée pour la circonstance.

Des lots de 6 rats mâles, pesant environ 150 g, mis à jeun et privés de boisson la veille de l'essai, ont été placés par deux dans des cages à métabolisme pour une durée de 6 heures.

Les produits à essayer ont été administrés par voie orale, à la sonde, en même temps qu'une surcharge hydrique (eau du robinet à 37 °C), à raison de 5 ml pour 100 g de poids corporel.

Les volumes urinaires ont été recueillis et mesurés toutes les heures, et la quantité d'urine excrétée est donnée en ml par heure et par rat.

Les dosages du chlore, du sodium et du potassium sont effectués sur les prélèvements de 6 heures et sont exprimés en Meq par litre d'urine et par 100 g de rat.

A partir de ces chiffres et par rapport aux témoins, des pourcentages d'augmentation ou de diminution de la diurèse ont pu être calculés.

L'excrétion urinaire est donnée par le rapport :

$$E.\ U. = \frac{\text{Vol. urinaire recueilli x 100}}{\text{Vol. moyen d'eau administré}}$$

L'activité diurétique est obtenue en faisant le rapport :

$$\frac{\text{E.U. traités}}{\text{E.U. témoins}}$$

Les résultats obtenus figurent dans les tableaux I à III ci-après ; ils permettent de constater que les composés A, B et C suivant l'invention, tout comme l'hydrochlorothiazide et le furosémide, augmentent la diurèse.

Dans le tableau I figure l'action diurétique avec surcharge hydrique pour les composés A, B et C, et pour le furosémide et l'hydrochlorothiazide.

Dans le tableau II figure l'action diurétique avec surcharge hydrosodée pour le composé A et le furosémide.

A titre d'exemple, à la dose de 10 mg/kg le composé A a une activité diurétique sensiblement égale à celle de 25 mg/kg de furosémide, ce qui correspond à une action diurétique sensiblement double.

D'autre part, aux doses équivalentes ci-dessus, la fuite de potassium pour le composé A (19 %) est très significativement inférieure à celle correspondant au furosémide (30,3 %).

La diurèse a d'autre part été confirmée chez le chien Beagle, par voie I.P. La diurèse observée au bout d'une heure en fonction de la dose injectée est représentée dans le tableau III où l'on constate que, pour le composé A, la diurèse maximale au bout d'une heure est atteinte avec une dose de 2 mg/kg et que la diurèse est multipliée par 600/100. Alors que le sodium éliminé dans le volume d'urine émis est voisin de 10 fois celui du témoin, le taux de potassium n'est multiplié que par 1,2.

TABLEAU I

ACTION DIURETIQUE EN SURCHARGE HYDRIQUE SUR LE RAT

| Dose adminis-trée en mg/kg | Composé A | | | Composé B | | | Composé C | | | Furosémide | | | Hydrochloro-thiazide | | |
|---|---|---|---|---|---|---|---|---|---|---|---|---|---|---|---|
| | dV | Na | K | dV | Na | K | dV | Na | K | dV | Na | K | dV | Na | K |
| 0,1 | + 4,4 | + 84,4 | +19 | + 4 | + 73 | + 8 | + 8 | + 57 | +15 | - | - | - | + 1 | +6,9 | + 1 |
| 1 | + 8,8 | +139,4 | +18 | + 5 | +160 | +15 | +15 | +141 | +17 | - | - | - | +38 | +374 | +29 |
| 10 | +38,3 | +551 | +71 | +30 | +582 | +35 | +35 | +513 | +47 | - | - 22 | -19 | +40 | +430 | +22 |
| 25 | +39,7 | +672 | +67 | +35 | +671 | +33 | +31 | +583 | +42 | +18 | + 32 | + 2 | +48 | +504 | +44 |
| 50 | +23,6 | +606 | +90 | +31 | +550 | +21 | +18 | +531 | +57 | +76 | +639 | +84 | | | |

dV = pourcentage du volume d'urine émise par rapport au lot témoin et ramené à 100 g de poids corporel

Na = nbre de meq/L pour 100 g d'animal

K = nbre de meq/L pour 100 g d'animal

Composé A = Chloro-7 diméthylamino-3 dioxo-2,4 (1H,3H) sulfonamido-6 quinazoline

Composé B = Chloro-7 diéthylamino-3 dioxo-2,4 (1H,3H) sulfonamido-6 quinazoline

Composé C = Chloro-7 pipéridino-3 dioxo-2,4 (1H,3H) sulfonamido-6 quinazoline.

TABLEAU II

ACTION DIURETIQUE EN SURCHARGE HYDROSODEE SUR LE RAT

| Dose Administrée en mg/kg | Composé A | | | Furosémide | | |
|---|---|---|---|---|---|---|
| | dV | Na | K | dV | Na | K |
| 1 | - 11 | - 1 | - 13 | | | |
| 5 | - | - | - | - 30 | - 24 | - 15 |
| 10 | + 27 | + 43 | + 18 | + 23 | + 23 | + 30 |
| 25 | + 56 | + 64 | + 38 | | | |
| 50 | + 73 | + 75 | + 30 | | | |

dV = pourcentage du volume d'urine émise par rapport au lot témoin et ramené à 100 g de poids corporel

Na = nombre de meq/L pour 100 g d'animal

K = nombre de meq/L pour 100 g d'animal

TABLEAU III

## DIURESE CHEZ LE CHIEN BEAGLE

| Dose I.V. mg/kg | Emission d'urine en ml | | | | | | | |
|---|---|---|---|---|---|---|---|---|
| | 0,5 mn | 5-10 mn | - 20 mn | - 30 mn | - 40 mn | - 50 mn | - 60mn | TOTAL |
| Témoin | 2,0 | 2,0 | 1,0 | 1,5 | 3,5 | 3,5 | 3,0 | 16,5 |
| 1 | 3,5 | 5,0 | 8,5 | 7,0 | 9,0 | 8,0 | 9,0 | 50,0 |
| 2 | 8,0 | 14,5 | 20,5 | 18,5 | 15,5 | 11,5 | 8,5 | 97,0 |
| 5 | 9,5 | 10,5 | 10,0 | 8,0 | 9,0 | 5,5 | 7,0 | 59,5 |
| 10 | 4,0 | 8,0 | 9,0 | 10,0 | 4,0 | 8,0 | 7,5 | 50,5 |

Les composés de l'invention sont notamment administrables par voie orale et parentérale.

Pour ces modes d'administration, le médicament est avantageusement formulé en doses unitaires telles que comprimés, gélules, solutions injectables, etc...dans lesquelles le principe actif est associé aux excipients et véhicules pharmaceutiques usuels appropriés.

Chaque dose unitaire contient avantageusement de 5mg à 30 mg de principe actif.

On donnera ci-dessous, à titre d'exemple, deux formulations du médicament de l'invention.

Comprimés :

chloro-7 diméthylamino-3 dioxo-2,4 sulfamido-6 (1H,3H) quinazoline..................................15 à 20 mg

excipient pour un comprimé.

Soluté injectable :

- Chloro-7 diméthylamino-3 dioxo-2,4 sulfamido-6 (1H, 3H) quinazoline ...................10 mg
- Chlorure de sodium.................15 mg
- Eau distillée : q.s. pour une ampoule de 2 ml.

## REVENDICATIONS

1 - Composés de formule :

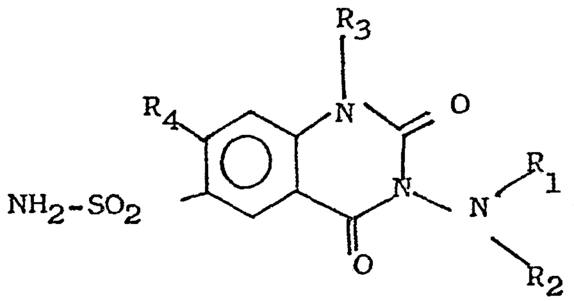

(I)

dans laquelle $R_1$ représente l'hydrogène ou un groupe alkyle en $C_1$ à $C_5$ et $R_2$ représente un groupe alkyle inférieur, ou bien $R_1$ et $R_2$ forment ensemble et avec l'atome d'azote auquel ils sont fixés un hétérocycle à 5 ou 6 chaînons pouvant contenir un autre hétéroatome ; $R_3$ représente l'hydrogène ou un groupe alkyle inférieur et $R_4$ représente l'hydrogène ou un atome d'halogène ; la forme tautomère de type amino-3 hydroxy-2 (3,H) quinazoline-4 des composés de formule I dans lesquels $R_3$ est l'hydrogène ; et les sels d'addition avec des acides de composés ci-dessus.

2 - Composés suivant la revendication 1 dans lesquels $R_3$ est un atome d'hydrogène et $R_4$ est un atome d'halogène.

3 - Composé suivant la revendication 1 qui est la diméthylamino-3 dioxo-2,4 (1H, 3H) sulfonamido-6 quinazoline; la chloro-7 diméthylamino-3 dioxo-2,4 (1H,3H) sulfonamido-6 quinazoline ; la méthyl-1 chloro-7 diméthylamino-3 dioxo-2,4 (1H, 3H) sulfonamido-6 quinazoline ; la méthyl-1 diméthylamino-3 dioxo-2,4 (1H,3H) sulfonamido-6 quinazoline ; la chloro-7 morpholino-3 dioxo-2,4 (1H,3H) sulfonamido-6 quinazoline ; la méthyl-1 chloro-7 morpholino-3 dioxo-2,4 (1H,3H) sulfonamido-6 quinazoline ; l'éthyl-1 chloro-7 diméthylamino-3 dioxo-2,4 (1H,3H) sulfonamido-6 quinazoline ; la chloro-7 diéthylamino-3 dioxo-2,4 (1H,3H)

sulfonamido-6 quinazoline ; la chloro-7 pipéridino-3 dioxo-2,4 (1H, 3H) sulfonamido-6 quinazoline ; la méthyl-1 chloro-7 pipéridino-3 dioxo-2,4 sulfonamido-6 quinazoline; ou la chloro-7 N-méthylpipérazino-3 dioxo-2,4 sulfonamido-6 quinazoline.

4 - Procédé de préparation des composés suivant la revendication 1, caractérisé en ce qu'on fait réagir de l'acide chlorosulfonique sur une amino-3 (1H,3H) dioxo-2,4 quinazoline de formule :

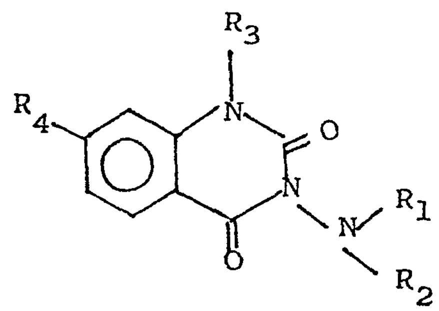

(II)

dans laquelle $R_1$, $R_2$, $R_3$ et $R_4$ ont les significations données à la revendication 1, obtenant ainsi un composé de formule :

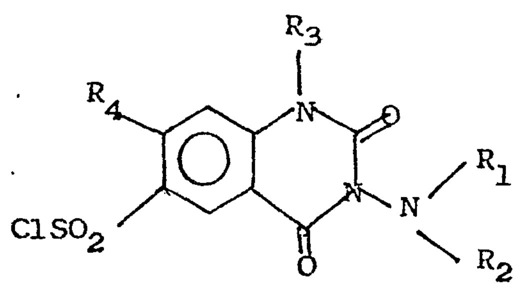

(III)

dans laquelle $R_1$, $R_2$, $R_3$ et $R_4$ ont les significations données à la revendication 1, que l'on fait ensuite réagir avec de l'ammoniac pour obtenir le composé de formule (I)

5 - Composition thérapeutique ayant notamment une activité diurétique, caractérisée en ce qu'elle contient, à titre de principe actif, un composé suivant la revendication 1 ou 2 ou un sel d'addition avec un acide thérapeutiquement administrable de celui-ci, et un véhicule pharmaceutiquement acceptable.

6 - Composition thérapeutique suivant la revendication 5, caractérisée en ce qu'elle est sous forme de doses unitaires pour l'administration orale ou parentérale.

0012051

Office européen des brevets

# RAPPORT DE RECHERCHE EUROPEENNE

Numéro de la demande

EP 79 40 0838

| DOCUMENTS CONSIDERES COMME PERTINENTS | | | CLASSEMENT DE LA DEMANDE (Int. Cl. 3) |
|---|---|---|---|
| Catégorie | Citation du document avec indication, en cas de besoin, des parties pertinentes | Revendication concernée | |
| D | CHEMICAL ABSTRACTS, vol. 79 (1973) page 457, abstract 18752n Columbus, Ohio, USA<br>& JP - A - 73 01674 (EISAI CO.LTD.) 19 janvier 1973.<br>* Résumé *<br>---- | 1,2,5, 6 | C 07 D 239/96<br>401/04<br>403/04<br>413/04<br>A 61 K 31/505 |

DOMAINES TECHNIQUES RECHERCHES (Int. Cl. 3)

C 07 D 239/96
401/04
403/04
413/04
A 61 K 31/505

CATEGORIE DES DOCUMENTS CITES

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

X Le présent rapport de recherche a été établi pour toutes les revendications

| Lieu de la recherche | Date d'achèvement de la recherche | Examinateur |
|---|---|---|
| La Haye | 24-01-1980 | FRANCOIS |